## Europäisches Patentamt

## European Patent Office

## Office européen des brevets

(19)

(11) **EP 1 174 114 B1**

(12) **FASCICULE DE BREVET EUROPEEN**

(45) Date de publication et mention de la délivrance du brevet:
**09.11.2005 Bulletin 2005/45**

(51) Int Cl.⁷: **A61K 7/06**, A61K 7/00

(21) Numéro de dépôt: **01401838.6**

(22) Date de dépôt: **10.07.2001**

(54) **Procédé d'éclaircissement ou de teinture temporaire des cheveux, et dispositif aérosol permettant de mettre en oeuvre ce procédé**

Verfahren zur nichtpermanenten Haaraufhellung oder Haarfärbung, und Aerosolvorrichtung zur durchführung des Verfahrens

Method of temporarly lightening or coloring hair, and aerosol device for performing the same

(84) Etats contractants désignés:
**AT BE CH CY DE DK ES FI FR GB GR IE IT LI LU MC NL PT SE TR**

(30) Priorité: **18.07.2000 FR 0009407**

(43) Date de publication de la demande:
**23.01.2002 Bulletin 2002/04**

(73) Titulaire: **L'OREAL**
**75008 Paris (FR)**

(72) Inventeurs:
• **Giroud, Franck**
**92110 Clichy (FR)**

• **Samain, Henri**
**91570 bièvres (FR)**

(74) Mandataire: **Casalonga, Axel et al**
**BUREAU D.A. CASALONGA - JOSSE**
**Paul-Heyse-Strasse 33**
**80336 München (DE)**

(56) Documents cités:
**EP-A- 0 761 199        EP-A- 0 919 219**
**EP-A- 1 004 288        WO-A-98/25710**
**US-A- 4 172 122        US-A- 5 593 680**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne un procédé d'éclaircissement et/ou de teinture temporaire des cheveux par vaporisation d'une solution d'un polymère formant après évaporation du solvant un dépôt opaque de couleur claire et un dispositif aérosol permettant de mettre en oeuvre ce procédé.

La décoloration des fibres kératiniques humaines, en particulier des cheveux, se fait généralement par oxydation de la mélanine aboutissant à la solubilisation et à l'élimination partielle ou totale de ce pigment.

Ce traitement chimique par des agents oxydants et alcalins est souvent très agressif et modifie la structure chimique de la kératine entraînant ainsi une dégradation des fibres kératiniques et une altération des propriétés cosmétiques des cheveux.

La mise en oeuvre des traitements éclaircissants oxydants est par ailleurs assez longue et complexe. Le caractère définitif de l'éclaircissement obtenu peut paraître, à première vue, avantageux. Toutefois, la durabilité de l'effet implique, lors de la repousse des cheveux, le renouvellement du traitement à des intervalles réguliers ce qui rend ce type de traitement assez contraignant.

Un traitement éclaircissant, pratique d'emploi et n'induisant pas de dégradation des fibres kératiniques constituerait par conséquent un progrès considérable dans le domaine des traitements capillaires.

La demanderesse a découvert, de manière assez inattendue, que l'application sur les cheveux de certains polymères thermofusibles cristallins sous forme d'aérosol, donnait lieu à la formation de dépôts polymériques opaques de couleur claire.

L'effet éclaircissant ainsi obtenu est temporaire car le dépôt polymérique opaque peut être éliminé par un simple lavage des cheveux. La fugacité de cet éclaircissement constitue un avantage. En effet, la simplicité du traitement et l'absence totale de dégradation des fibres kératiniques n'interdisant plus des applications multiples, le procédé d'éclaircissement de la présente invention permet une grande liberté d'utilisation. L'utilisateur peut ainsi faire varier à souhait l'intensité et la localisation des zones éclaircies.

Par ailleurs, l'adjonction de colorants ou de pigments à la solution éclaircissante contenant ledit polymère thermofusible cristallin permet d'obtenir des nuances colorées que l'utilisateur pourra associer en toute liberté entre elles ou à des éléments de sa garderobe.

La présente invention a pour objet un dispositif aérosol pour l'éclaircissement et/ou la coloration temporaire des cheveux, lequel dispositif est constitué

- d'un récipient contenant une composition aérosol formée par un composé propulseur et une phase liquide qui est une solution éclaircissante contenant, à l'état dissous dans un milieu cosmétiquement acceptable au moins un polymère thermofusible cristallin ayant un point de fusion cristalline, mesuré par analyse enthalpique différentielle, compris entre 30 et 80 °C, et au moins un solvant volatil cosmétiquement acceptable ayant une température d'ébullition inférieure à 90 °C, et

- d'un moyen de distribution de la dite composition aérosol, approprié pour obtenir un débit de matière sèche inférieur ou égal à 12 mg/s et un pouvoir mouillant, à 20 cm, inférieur ou égal à 100 mg/s, approprié pour permettre l'obtention, après évaporation du solvant, d'un dépôt opaque de couleur claire.

La présente invention a également pour objet un procédé d'éclaircissement et/ou de coloration temporaire des cheveux caractérisé par le fait que l'on applique sur les cheveux secs par vaporisation une solution éclaircissante contenant, à l'état dissous dans un milieu cosmétiquement acceptable, au moins polymère thermofusible cristallin ayant un point de fusion cristalline, mesuré par analyse enthalpique différentielle, compris entre 30 et 80°C et au moins un solvant volatil cosmétiquement acceptable ayant une température d'ébullition inférieure à 90°C, de manière à former, après évaporation du solvant, un dépôt opaque de couleur claire et par le fait que l'application se fait au moyen d'un dispositif aérosol selon la présente invention.

La présente invention permet ainsi par simple vaporisation d'une solution éclaircissante sur les cheveux secs, par exemple au moyen du dispositif aérosol décrit plus en détail ci-dessous, d'obtenir, en l'espace de quelques dizaines de secondes après évaporation du solvant, un dépôt clair plus ou moins opaque, éventuellement coloré. Le revêtement ainsi déposé sur les cheveux est dépourvu d'effet de "poudrage", c'est-à-dire il ne présente pas l'aspect d'une poudre pulvérisée sur les cheveux et résiste bien aux sollicitations mécaniques.

Les polymères thermofusibles cristallins utilisables selon la présente invention pour l'éclaircissement et/ou la coloration temporaire des cheveux sont de préférence des copolymères cristallins comprenant

i) de 85 à 98 % en poids de motifs hydrophobes et
ii) de 2 à 15 % en poids de motifs hydrophiles.

Les motifs hydrophobes sont dérivés de monomères $\alpha,\beta$-éthyléniques à chaîne latérale n-alkyle en $C_{12-50}$, de préférence en $C_{14-24}$, formant des homopolymères cristallins appelés dans la littérature anglo-saxonne *side chain crystalline polymers*. Il s'agit en particulier d'acrylates et de méthacrylates de n-alkyle en $C_{12-50}$ et de préférence en $C_{14-24}$.

Les motifs hydrophiles sont dérivés de préférence d'acides monocarboxyliques en $C_{3-6}$ $\alpha,\beta$-insaturés tels que l'acide acrylique, l'acide méthacrylique ou l'acide crotonique, d'acides dicarboxyliques insaturés

en C$_{4-6}$ tels que l'acide maléique et l'acide itaconique, ou des esters et amides à chaîne alkyle en C$_{1-4}$ de ces acides monocarboxyliques ou dicarboxyliques tels que les (méth)acrylates d'alkyle en C$_{1-4}$ et les N-(alkyle en C$_{1-4}$)-(méth)acrylamides.

La synthèse de ces polymères est décrite notamment dans la demande de brevet internationale WO 98/25710.

On peut également utiliser comme motifs hydrophiles le méthacrylate d'hydroxyéthyle ou la vinylpyrrolidone.

Les groupements acide carboxylique des motifs hydrophiles sont de préférence partiellement ou totalement neutralisés par une base choisie par exemple parmi la soude, la potasse, l'amino-2-méthyl-2-propanol, le monoéthanolamine, le triéthanolamine ou la triisopropanolamine.

Les polymères thermofusibles cristallins utilisables dans la présente invention sont par ailleurs caractérisés par un point de fusion cristalline relativement peu élevé, c'est-à-dire compris entre 30 et 80 °C. Ce point de fusion relativement bas permet une élimination facile des polymères par simple lavage des cheveux avec de l'eau ayant une température supérieure au point de fusion du polymère.

Le point de fusion cristalline des polymères utilisables dans la présente invention est mesuré par analyse enthalpique différentielle (en anglais : *Differential Scanning Calorimetry*).

L'enthalpie de fusion d'un polymère est la quantité d'énergie nécessaire pour transformer un échantillon partiellement ou totalement cristallin en un échantillon totalement amorphe. Le thermogramme $\Delta Cp = f(T)$, dans lequel $\Delta Cp$ représente la différence de capacité thermique de l'échantillon par rapport à un échantillon de référence ne subissant aucune transition thermique dans le domaine étudié, présente donc un signal endothermique dont l'aire est proportionnelle à l'enthalpie de fusion de l'échantillon.

Le point de fusion des polymères cristallins utilisables dans la présente invention est mesuré à l'aide d'un appareil d'analyse enthalpique différentielle (DSC), modèle M2920CE-5010 commercialisé par la société TA Instruments. On chauffe l'échantillon, à une vitesse de 10 °C/minute, de -20 °C à + 150 °C, et on enregistre la différence de capacité thermique entre l'échantillon et le témoin en fonction de la température.

On appelle point de fusion cristalline (T$_f$) la température correspondant au sommet du pic endothermique de fusion des zones cristallines ainsi obtenu.

On peut citer à titre d'exemple de polymères cristallins à bas point de fusion tels que ceux décrits ci-dessus, un copolymère commercialisé sous la dénomination STRUCTURE® O par la société NATIONAL STARCH. Il s'agit d'un copolymère statistique constitué de 10 % en poids de motifs dérivés d'acide acrylique et de 90 % en poids de motifs dérivés de méthacrylate de n-octadécyle. Ce copolymère a un point de fusion cristalline, mesuré par analyse enthalpique différentielle, égal à 46 °C.

Le ou les polymères cristallins thermofusibles décrits ci-dessus sont appliqués sur les cheveux sous forme dissoute dans un solvant ou mélange de solvants cosmétiquement acceptable, suffisamment volatil pour permettre un séchage rapide, c'est-à-dire de l'ordre de quelques dizaines de secondes, du dépôt déposé par vaporisation.

On utilise de préférence des solvants ayant un point d'ébullition, à pression atmosphérique, inférieur ou égal à 90 °C. On peut citer à titre d'exemple de solvants utilisables dans la présente invention les alcools en C$_{1-4}$ tels que l'éthanol ou l'isopropanol, l'acétone, la méthyléthylcétone, l'acétate de vinyle, l'acétate de méthyle, le diméthoxyéthane et des mélanges de ceux-ci. Parmi ces solvants, on utilise de préférence l'éthanol.

La solution éclaircissante peut également contenir en plus une fraction d'un ou de plusieurs solvants cosmétiquement acceptables ayant une température d'ébullition supérieure à 90 °C, tels que l'eau, les alcanes en C$_{6-10}$, le diéthoxyéthane et l'acétate d'éthyle.

Dans un mode de réalisation préféré de la présente invention, ce solvant ou mélange de solvants, cosmétiquement acceptable, représente au moins 35 % en poids de la solution éclaircissante.

La concentration dudit polymère thermofusible cristallin est de préférence comprise entre 0,1 et 35 % en poids, de préférence comprise entre 0,1 et 6 % en poids, rapporté au poids total de la dite solution éclaircissante.

Dans un mode de réalisation particulier des compositions et du procédé de la présente invention, la solution éclaircissante contient en outre un ou plusieurs colorants ou pigments. Ces colorants ou pigments sont choisis parmi ceux indiqués par exemple dans l'ouvrage de Charles Zviak, Science des traitements capillaires, Masson, (1988), chapitre 7, ou dans le Color Index International, 3$^{ème}$ édition.

La solution éclaircissante peut contenir en outre un ou plusieurs additifs ou adjuvants utilisés habituellement dans des compositions capillaires. Ces additifs ou adjuvants sont choisis notamment parmi les épaississants, les tensioactifs anioniques, non ioniques cationiques ou amphotères, les parfums, les conservateurs, les filtres solaires, les protéines, les vitamines, les provitamines, les polymères non fixants anioniques non ioniques, cationiques ou amphotères, les huiles minérales, végétales ou synthétiques, les céramides, les pseudocéramides, les silicones volatiles ou non, linéaires ou cycliques, modifiées ou non modifiées, et tout autre additif classiquement utilisé dans les compositions cosmétiques destinées à être appliquées sur les cheveux.

Le composé propulseur est choisi parmi les gaz comprimés ou liquéfiés usuellement employés pour la préparation de compositions aérosols. On utilisera de manière préférentielle l'air, le gaz carbonique ou l'azote comprimés, ou encore un gaz soluble ou non dans la composition, tel que l'éther diméthylique, les hydrocarbures, les hydrocarbures halogénés , en particuliers fluorés, et des mélanges de ces composés.

Le moyen de distribution est généralement une valve de distribution commandée par une tête de distribution comprenant une buse par laquelle la composition aérosol est vaporisée.

On entend par "débit en matière sèche" selon la présente invention la quantité d'extrait sec sortant du dispositif aérosol par unité de temps. Ce débit en matière sèche est exprimé en mg/s et est égal au produit de la concentration en matière sèche de la composition aérosol ($C_{MS}$) par le débit en composition aérosol à la sortie de la buse ($D_{CA}$) :

$$D_{MS} = C_{MS} \times D_{CA}$$

La concentration en matière sèche de la composition aérosol, exprimée en %, est égale à la quantité (en g) de matière sèche rapportée à 100 g de composition aérosol (phase liquide + composé propulseur). La quantité de matière sèche est déterminée après séchage du résidu de pulvérisation pendant 1 heure 30 à une température de 105 °C.

Le débit en composition aérosol ($D_{CA}$) correspond à la quantité de composition aérosol (phase liquide + propulseur) sortant du dispositif de distribution par unité de temps. On détermine ce débit en composition aérosol, exprimé en mg/s, en mesurant la différence entre le poids de l'aérosol avant ($M_0$) et après ($M_1$) 10 secondes de vaporisation et en rapportant cette différence à 1 seconde :

$$D_{CA} = (M_0 - M_1) / 10$$

Selon la présente invention le "pouvoir mouillant" correspond à la quantité de produit reçue par une feuille de matière plastique disposée à une distance de 20 cm de la buse du dispositif aérosol pendant un laps de temps donné. Le produit reçu par la feuille est constitué de matière sèche, de solvant non encore évaporé et éventuellement de composé propulseur mélangé à la phase liquide.

Le pouvoir mouillant, exprimé en mg/s, est mesuré de la manière suivante :

On suspend verticalement une feuille de matière plastique de dimensions 21 cm x 23 cm à une balance de précision (1/1000), la feuille étant fixée à la balance par le bord supérieur (généralement par un crochet de la balance inséré dans une perforation disposée au centre de la largeur de la feuille et à 1 cm du bord supérieur de celle-ci) et étant maintenue en position verticale par l'application d'un poids centré sur le bord inférieur (généralement par une pince fixée et centrée sur le bord inférieur).

On place ensuite une cale derrière le bord inférieur de la feuille pour maintenir la feuille verticale lors de l'impact du produit.

On dispose verticalement le dispositif aérosol de manière à ce que la buse de diffusion de la composition soit disposée au centre et à une distance de 20 cm de la feuille verticale pour permettre une vaporisation du produit perpendiculaire à la feuille.

On vaporise la composition pendant 5 secondes.

On mesure la quantité de produit reçue par la feuille dès la fin de la vaporisation.

Pour plus de précision, on peut employer un dispositif approprié comprenant un moyen de support du dispositif aérosol, et des moyens permettant le réglage tridimensionnel de la position de la buse par rapport à la feuille verticale. Ce dispositif peut être également équipé d'un dispositif pneumatique de contrôle du temps de vaporisation, de manière à contrôler avec précision la durée de la vaporisation. L'ensemble peut être contrôlée par un ordinateur.

Pour éviter les perturbations provenant de l'environnement immédiat, le trajet du produit entre la buse et la feuille sera avantageusement protégé par les parois d'un tunnel de dimensions appropriées.

Enfin, la vaporisation du produit est avantageusement effectuée sous atmosphère contrôlée, de manière préférentielle à une température de 20 °C et une humidité relative de 50 %.

Le débit en matière sèche et le pouvoir mouillant à 20 cm du dispositif aérosol permettre le dépôt d'une quantité suffisante de solution éclaircissante.

Selon un mode de réalisation préféré de l'invention, le dispositif aérosol est approprié pour l'obtention d'un débit en matière sèche compris entre 2 et 12 mg/s.

Selon un autre mode de réalisation préféré de l'invention, le dispositif aérosol est approprié pour l'obtention d'un pouvoir mouillant, à 20 cm, compris entre 10 et 100 mg/s, et de préférence entre 20 et 80 mg/s.

Le débit en matière sèche et le pouvoir mouillant du dispositif aérosol de l'invention dépendent, d'une part, de la composition aérosol et, d'autre part, du moyen de distribution, les deux devant être appropriés pour obtenir l'effet recherché. Parmi les paramètres susceptibles d'influencer ces caractéristiques, on peut citer plus particulièrement la concentration en matière sèche ($C_{MS}$) de la phase liquide, le débit en composition aérosol ($D_{CA}$) et le rapport en poids phase liquide/composé propulseur.

La concentration en matière sèche est avantageusement inférieure ou égale à 25 % en poids par rapport au poids total de la composition aérosol (phase liquide + composé propulseur), de préférence comprise entre 0,4 et 5 % en poids et en particulier entre 0,6 et 3,25 % en poids.

Le débit en composition aérosol est de préférence supérieur ou égal à 200 mg/s, mieux encore comprise entre 200 et 600 mg/s et plus préférentiellement entre 300 et 400 mg/s.

La composition aérosol est de préférence une "phase longue" c'est-à-dire le rapport en poids phase liquide/composé propulseur est de préférence supérieur à 1 et plus particulièrement compris entre 1,2 et 3.

L'homme du métier saura choisir en fonction de la composition aérosol le moyen de distribution approprié pour obtenir les caractéristiques de débit en matière sèche et de pouvoir mouillant recherchées.

Les valves appropriées pour les compositions particulières ci-dessus sont notamment des valves décrites dans le brevet FR 2 382 637 (Abplanalp) et commercialisées sous la dénomination AQUASOL® par la Société PRECISION. Ces valves comportent des conduits séparés destinés aux produits liquides et au composé propulseur communiquant par une chambre de mélange par choc du type éjecteur de Venturi dans laquelle les flux de produit et de composé propulseur sont introduits librement et entrent en collision et se mélangent pour former une dispersion fine qui est éjectée par un orifice. Les valves comportent également des éléments de distribution pouvant être manoeuvrés simultanément par un seul organe commandant l'ensemble des flux.

Les valves particulièrement appropriées pour la présente invention comportent :

- un gicleur présentant au moins un orifice ayant un diamètre compris entre 0,40 mm et 0,60 mm, de préférence deux orifices et plus particulièrement deux orifices ayant chacun un diamètre de 0,5 mm ;
- une restriction interne du corps de valve comprise entre 0,3 mm et 1 mm, de préférence comprise entre 0,3 mm et 0,6 mm ;
- un orifice de buse ayant un diamètre compris entre 0,3 et 1 mm, de préférence entre 0,3 et 0,6 mm ;
- un élément en forme de disque à deux canaux de dimensions comprises entre 0,25 mm x 0,25 mm et 0,45 mm x 0,54 mm, de préférence égales à 0,25 mm x 0,25 mm.

On utilisera de préférence une valve avec un gicleur à deux orifices ayant chacun un diamètre de 0,5 mm, un corps de valve avec une restriction de 0,36 ou , 58 mm, un orifice de buse ayant respectivement un diamètre de 0,36 mm ou 0,60 mm, et un élément en forme de disque à deux canaux de dimensions 0,25 mm x 0,25 mm.

Les diffuseurs appropriés pour les compositions de la présente invention sont en particulier les boutons poussoir à buse tourbillonaire, tels que celui commercialisé par la société PRECISION sous la référence 216943-40.

Les exemples ci-après permettent d'illustrer l'invention sans toutefois en limiter la portée.

**Exemple 1**

On solubilise dans un mélange d'heptane et d'éthanol (80/20 en poids) le polymère STRUCTURE O à une concentration de 40 g/litre.

On introduit 65 g de cette solution éclaircissante dans un récipient aérosol muni d'une valve (V36) avec un gicleur à deux orifices ayant chacun un diamètre de 0,5 mm, un corps de valve avec une restriction de 0,36, un orifice de buse ayant un diamètre de 0,36 mm, et un élément en forme de disque à deux canaux de dimensions 0,25 mm x 0,25 mm. On ajoute ensuite 35 g d'éther diméthylique comme propulseur et on adapte un bouton poussoir commercialisé sous la référence 216943-40 par la société PRECISION.

Avec ce dispositif aérosol, on obtient un débit en matière sèche de 6,5 mg/s et un pouvoir mouillant, à 20 cm, mesuré dans les conditions décrites ci-avant, égal à 26 mg/s.

On pulvérise la solution éclaircissante pendant 10 secondes sur des cheveux naturels secs de couleur châtain. Dix secondes après la pulvérisation, les cheveux apparaissent éclaircis.

**Exemple 2**

On prépare une solution éclaircissante colorée en ajoutant à la solution éclaircissante de l'Exemple 1 0,1 % en poids d'un colorant bleu (Dianix Blue F2B-SE de la société DYSTAR) parfaitement soluble dans le mélange de solvant.

On introduit cette solution éclaircissante colorée dans un dispositif aérosol identique à celui décrit dans l'Exemple 1 et on la pulvérise pendant 10 secondes sur des cheveux naturels secs de couleur châtain. Environ 10 secondes après la pulvérisation, les cheveux apparaissent éclaircis et possèdent un reflet bleu.

**Revendications**

1. Dispositif aérosol pour l'éclaircissement et/ou la coloration temporaire des cheveux, constitué

   - d'un récipient contenant une composition aérosol formée par un composé propulseur et une phase liquide, et
   - d'un moyen de distribution de ladite composition aérosol,

   **caractérisé par le fait que** ladite phase liquide est une solution éclaircissante contenant, à l'état dissous dans un milieu cosmétiquement acceptable, au moins polymère thermofusible cristallin ayant un point de fusion cristalline, mesuré par analyse enthalpique différentielle, compris entre 30 et 80°C et au moins un solvant volatil cosmétiquement acceptable ayant une température d'ébullition inférieure à 90°C, et **par le fait que** le moyen de distribution, approprié pour obtenir un débit de matière sèche inférieur ou égal à 12 mg/s et un pouvoir mouillant, à 20 cm, inférieur ou égal à 100 mg/s, est approprié pour permettre l'obtention, après évaporation du solvant, d'un dépôt opaque de couleur claire.

**2.** Dispositif aérosol selon la revendication 1, **caractérisé par le fait que** ledit moyen de distribution est approprié pour obtenir un débit en matière sèche compris entre 2 et 12 mg/s.

**3.** Dispositif aérosol selon la revendication 1, **caractérisé par le fait que** ledit moyen de distribution est approprié pour obtenir un pouvoir mouillant compris entre 10 et 100 mg/s, de préférence entre 20 et 80 mg/s.

**4.** Dispositif aérosol selon l'une quelconque des revendications 1 à 3, **caractérisé par le fait que** ledit moyen de distribution permet d'obtenir un débit en composition aérosol supérieur à 200 mg/s, de préférence compris entre 200 et 600 mg/s, et en particulier compris entre 300 et 400 mg/s.

**5.** Dispositif aérosol selon l'une quelconque des revendications 1 à 4, **caractérisé par le fait que** la concentration en matière sèche de la composition aérosol (solution éclaircissante + composé propulseur) est inférieure ou égale à 25 % en poids, de préférence comprise entre 0,4 et 5 % en poids et en particulier comprise entre 0,6 et 3,25 % en poids.

**6.** Dispositif aérosol selon l'une quelconque des revendications 1 à 5, **caractérisé par le fait que** le rapport pondéral solution éclaircissante/composé propulseur est supérieur à 1, de préférence compris entre 1,2 et 3.

**7.** Dispositif aérosol selon l'une quelconque des revendications 1 à 6, **caractérisé par le fait que** ledit polymère cristallin thermofusible est constitué

     i) de 85 à 98 % en poids de motifs hydrophobes dérivés de monomères $\alpha,\beta$-éthyléniques à chaîne latérale n-alkyle en $C_{12-50}$, de préférence en $C_{14-24}$, formant des homopolymères cristallins et
     ii) de 2 à 15 % en poids de motifs hydrophiles dérivés d'acides monocarboxyliques en $C_{3-6}$ $\alpha$, $\beta$-insaturés, d'acides dicarboxyliques insaturés en $C_{4-6}$, des esters et amides à courte chaîne de ces acides monocarboxyliques ou dicarboxyliques, le méthacrylate d'hydroxyéthyle et la vinylpyrrolidone.

**8.** Dispositif aérosol selon la revendication 7, **caractérisé par le fait que** ledit polymère cristallin thermofusible est un copolymère statistique contenant environ 10 % en poids de motifs dérivés d'acide acrylique et environ 90 % en poids de motifs dérivés de méthacrylate d'octadécyle.

**9.** Dispositif aérosol selon l'une quelconque des revendications 1 à 8, **caractérisé par le fait que** la concentration dudit polymère thermofusible cristallin est comprise entre 0,1 et 35 % en poids rapporté au poids total de la dite solution éclaircissante.

**10.** Dispositif aérosol selon l'une quelconque des revendications 1 à 9, **caractérisé par le fait que** ledit solvant volatil cosmétiquement acceptable ayant une température d'ébullition inférieure à 90 °C (a) est choisi parmi les alcools en $C_{1-4}$ tels que l'éthanol ou l'isopropanol, l'acétone, la méthyléthylcétone, l'acétate de vinyle, l'acétate de méthyle, le diméthoxyéthane et des mélanges de ceux-ci.

**11.** Dispositif aérosol selon l'une quelconque des revendications 1 à 10, **caractérisé par le fait que** la solution éclaircissante contient en outre au moins un solvant cosmétiquement acceptable ayant un point d'ébullition supérieur à 90 °C tel que l'eau, les alcanes en $C_{6-10}$, le diéthoxyéthane et l'acétate d'éthyle.

**12.** Dispositif aérosol selon l'une quelconque des revendications 1 à 11, **caractérisé par le fait que** la fraction du ou des solvants cosmétiquement acceptables représente au moins 35 % en poids de la solution éclaircissante.

**13.** Dispositif aérosol selon l'une quelconque des revendications 1 à 12, **caractérisé par le fait que** la solution éclaircissante contient en outre un ou plusieurs colorants ou pigments.

**14.** Dispositif aérosol selon l'une quelconque des revendications 1 à 13, **caractérisé par le fait que** ladite solution éclaircissante contient en outre un ou plusieurs additifs et adjuvants choisis parmi les épaississants, les tensioactifs anioniques, non ioniques cationiques ou amphotères, les parfums, les conservateurs, les filtres solaires, les protéines, les vitamines, les provitamines, les polymères non fixants anioniques, non ioniques, cationiques ou amphotères, les huiles minérales, végétales ou synthétiques, les céramides, les pseudocéramides, les silicones volatiles ou non, linéaires ou cycliques, modifiées ou non modifiées.

**15.** Procédé d'éclaircissement et/ou de coloration temporaire des cheveux **caractérisé par le fait que** l'on applique sur les cheveux secs par vaporisation une solution éclaircissante contenant, à l'état dissous, dans un milieu cosmétiquement acceptable, au moins polymère thermofusible cristallin ayant un point de fusion cristalline, mesuré par analyse enthalpique différentielle, compris entre 30 et 80°C et au moins un solvant volatil cosmétiquement acceptable ayant une température d'ébullition inférieure à 90°C, de manière à former, après évaporation du solvant, un dépôt opaque de couleur claire et **par le fait que** l'application se fait au moyen d'un dispositif aérosol selon l'une des revendications 1 à 14.

16. Procédé selon la revendication 15, **caractérisé par le fait que** ledit polymère cristallin thermofusible est constitué

> i) de 85 à 98 % en poids de motifs hydrophobes dérivés de monomères $\alpha,\beta$-éthyléniques à chaîne latérale n-alkyle en $C_{12\text{-}50}$, de préférence en $C_{14\text{-}24}$, formant des homopolymères cristallins et
> ii) de 2 à 15 % en poids de motifs hydrophiles dérivés d'acides monocarboxyliques en $C_{3\text{-}6}$ $\alpha$, $\beta$-insaturés, d'acides dicarboxyliques insaturés en $C_{4\text{-}6}$, des esters et amides à courte chaîne de ces acides monocarboxyliques ou dicarboxyliques, le méthacrylate d'hydroxyéthyle et la vinylpyrrolidone.

17. Procédé selon la revendication 16, **caractérisé par le fait que** ledit polymère cristallin thermofusible est un copolymère statistique contenant environ 10 % en poids de motifs dérivés d'acide acrylique et environ 90 % en poids de motifs dérivés de méthacrylate d'octadécyle.

18. Procédé selon l'une quelconque des revendications 15 à 17, **caractérisé par le fait que** ledit solvant volatil cosmétiquement acceptable ayant une température d'ébullition inférieure à 90 °C est choisi parmi les alcools en $C_{1\text{-}4}$ tels que l'éthanol ou l'isopropanol, l'acétone, la méthyléthylcétone, l'acétate de vinyle, l'acétate de méthyle, le diméthoxyéthane et des mélanges de ceux-ci.

19. Procédé selon la revendication 18, **caractérisé par le fait que** ledit solvant volatil est l'éthanol.

20. Procédé selon l'une quelconque des revendications 15 à 19, **caractérisé par le fait que** la solution éclaircissante contient en outre au moins un solvant cosmétiquement acceptable ayant un point d'ébullition supérieur à 90°C tel que l'eau, les alcanes en $C_{6\text{-}10}$, le diéthoxyéthane et l'acétate d'éthyle.

21. Procédé selon l'une quelconque des revendications 15 à 20, **caractérisé par le fait que** la fraction du ou des solvants cosmétiquement acceptables représente au moins 35 % en poids de la solution éclaircissante.

22. Procédé selon l'une quelconque des revendications 15 à 21, **caractérisé par le fait que** la solution éclaircissante contient en outre un ou plusieurs colorants ou pigments.

23. Procédé selon l'une quelconque des revendications 15 à 22, **caractérisé par le fait que** ladite solution éclaircissante contient en outre un ou plusieurs additifs et adjuvants choisis parmi les épaississants, les tensioactifs anioniques, non ioniques cationiques ou amphotères, les parfums, les conservateurs, les filtres solaires, les protéines, les vitamines, les provitamines, les polymères non fixants anioniques non ioniques, cationiques ou amphotères, les huiles minérales, végétales ou synthétiques, les céramides, les pseudocéramides, les silicones volatiles ou non, linéaires ou cycliques, modifiées ou non modifiées.

**Patentansprüche**

1. Aerosolvorrichtung zur temporären Aufhellung und/oder Färbung der Haare, bestehend aus

   -   einem Behälter, der eine aus einer Treibmittelverbindung und einer flüssigen Phase gebildete Aerosolzusammensetzung enthält, und
   -   einem Mittel zum Verteilen der Aerosolzusammensetzung,

   **dadurch gekennzeichnet, daß** es sich bei der flüssigen Phase um eine Aufhellerlösung handelt, die in einem kosmetisch unbedenklichen Medium gelöst mindestens ein kristallines wärmeschmelzbares Polymer mit einem mittels Differentialkalorimetrie bestimmten Kristallschmelzpunkt zwischen 30 und 80°C und mindestens ein kosmetisch unbedenkliches flüchtiges Lösungsmittel mit einem Siedepunkt von weniger als 90°C enthält, und mit dem Verteilungsmittel, mit dem ein Trockensubstanzdurchsatz kleiner gleich 12 mg/s und ein Benetzungsvermögen bei 20 cm kleiner gleich 100 mg/s erhältlich sind, nach Verdampfen des Lösungsmittels eine hell gefärbte opake Ablagerung erhältlich ist.

2. Aerosolvorrichtung nach Anspruch 1, **dadurch gekennzeichnet, daß** mit dem Verteilungsmittel ein Trockensubstanzdurchsatz zwischen 2 und 12 mg/s erhältlich ist.

3. Aerosolvorrichtung nach Anspruch 1, **dadurch gekennzeichnet, daß** mit dem Verteilungsmittel ein Benetzungsvermögen zwischen 10 und 100 mg/s und vorzugsweise zwischen 20 und 80 mg/s erhältlich ist.

**4.** Aerosolvorrichtung nach einem der Ansprüche 1 bis 3, **dadurch gekennzeichnet, daß** mit dem Verteilungsmittel ein Aerosolzusammensetzungsdurchsatz von mehr als 200 mg/s, vorzugsweise zwischen 200 und 600 mg/s und insbesondere zwischen 300 und 400 mg/s erhältlich ist.

**5.** Aerosolvorrichtung nach einem der Ansprüche 1 bis 4, **dadurch gekennzeichnet, daß** die Trockensubstanzkonzentration der Aerosolzusammensetzung (Aufhellerlösung + Treibmittelverbindung) kleiner gleich 25 Gew.-% ist und vorzugsweise zwischen 0,4 und 5 Gew.-% und insbesondere zwischen 0,6 und 3,25 Gew.-% liegt.

**6.** Aerosolvorrichtung nach einem der Ansprüche 1 bis 5, **dadurch gekennzeichnet, daß** das Gewichtsverhältnis von Aufhellerlösung zu Treibmittelverbindung größer als 1 ist und vorzugsweise zwischen 1,2 und 3 liegt.

**7.** Aerosolvorrichtung nach einem der Ansprüche 1 bis 6, **dadurch gekennzeichnet, daß** das wärmeschmelzbare kristalline Polymer aus

> i) 85 bis 98 Gew.-% hydrophoben Einheiten, die sich von $\alpha,\beta$-ethylenischen Monomeren mit einer $C_{12-50}$-n-Alkylseitenkette und vorzugsweise einer $C_{14-24}$-n-Alkylseitenkette, die kristalline Homopolymere bilden, ableiten, und
> ii) 2 bis 15 Gew.-% hydrophilen Einheiten, die sich von $\alpha,\beta$-ungesättigten $C_{3-6}$-Monocarbonsäuren, ungesättigten $C_{4-6}$-Dicarbonsäuren, kurzkettigen Estern und Amiden dieser Mono- oder Dicarbonsäuren, Hydroxyethylmethacrylat und Vinylpyrrolidon ableiten,

> besteht.

**8.** Aerosolvorrichtung nach Anspruch 7, **dadurch gekennzeichnet, daß** es sich bei dem wärmeschmelzbaren kristallinen Polymer um ein statistisches Copolymer mit ungefähr 10 Gew.-% Einheiten, die sich von Acrylsäure ableiten, und ungefähr 90 Gew.-% Einheiten, die sich von Octadecylmethacrylat ableiten, handelt.

**9.** Aerosolvorrichtung nach einem der Ansprüche 1 bis 8, **dadurch gekennzeichnet, daß** die Konzentration des kristallinen wärmeschmelzbaren Polymers zwischen 0,1 und 35 Gew.-%, bezogen auf das Gesamtgewicht der Aufhellerlösung, liegt.

**10.** Aerosolvorrichtung nach einem der Ansprüche 1 bis 9, **dadurch gekennzeichnet, daß** das kosmetisch unbedenkliche flüchtige Lösungsmittel mit einem Siedepunkt von weniger als 90°C (a) unter $C_{1-4}$-Alkoholen wie Ethanol oder Isopropanol, Aceton, Methylethylketon, Vinylacetat, Essigsäuremethylester und Dimethoxyethan und Gemischen davon ausgewählt ist.

**11.** Aerosolvorrichtung nach einem der Ansprüche 1 bis 10, **dadurch gekennzeichnet, daß** die Aufhellerlösung außerdem auch noch mindestens ein kosmetisch unbedenkliches Lösungsmittel mit einem Siedepunkt von mehr als 90°C wie Wasser, $C_{6-10}$-Alkane, Diethoxyethan und Essigsäureethylester enthält.

**12.** Aerosolvorrichtung nach einem der Ansprüche 1 bis 11, **dadurch gekennzeichnet, daß** sich der Anteil des oder der kosmetisch unbedenklichen Lösungsmittel auf mindestens 35 Gew.-% der Aufhellerlösung beläuft.

**13.** Aerosolvorrichtung nach einem der Ansprüche 1 bis 12, **dadurch gekennzeichnet, daß** die Aufhellerlösung außerdem auch noch ein oder mehrere Farbmittel oder Pigmente enthält.

**14.** Aerosolvorrichtung nach einem der Ansprüche 1 bis 13, **dadurch gekennzeichnet, daß** die Aufhellerlösung außerdem auch noch ein oder mehrere Additive oder Adjuvantien, die unter Verdickungsmitteln, anionischen, nichtionischen, kationischen oder amphoteren Tensiden, Riechstoffen, Konservierungsmitteln, Sonnenschutzmitteln, Proteinen, Vitaminen, Provitaminen, anionischen, nichtionischen, kationischen oder amphoteren nichtfestigenden Polymeren, mineralischen, pflanzlichen oder synthetischen Ölen, Ceramiden, Pseudoceramiden und gegebenenfalls modifizierten linearen oder cyclischen flüchtigen oder nichtflüchtigen Silikonen ausgewählt sind, enthält.

**15.** Verfahren zur temporären Aufhellung und/oder Färbung der Haare, **dadurch gekennzeichnet, daß** man auf die trockenen Haare durch Verdampfen eine Aufhellerlösung, die in einem kosmetisch unbedenklichen Medium gelöst mindestens ein kristallines wärmeschmelzbares Polymer mit einem mittels Differentialkalorimetrie bestimmten Kristallschmelzpunkt zwischen 30 und 80°C und mindestens ein kosmetisch unbedenkliches flüchtiges Lösungsmittel mit einem Siedepunkt von weniger als 90°C enthält, aufbringt, wodurch man nach Verdampfen des Lösungsmittels eine hell gefärbte opake Ablagerung erhält, und das Aufbringen mit Hilfe einer Aerosolvorrichtung nach einem der Ansprüche 1 bis 14 vornimmt.

**16.** Verfahren nach Anspruch 15, **dadurch gekennzeichnet, daß** das wärmeschmelzbare kristalline Polymer aus

i) 85 bis 98 Gew.-% hydrophoben Einheiten, die sich von α,β-ethylenischen Monomeren mit einer $C_{12-50}$-n-Alkylseitenkette und vorzugsweise einer $C_{14-24}$-n-Alkylseitenkette, die kristalline Homopolymere bilden, ableiten, und

ii) 2 bis 15 Gew.-% hydrophilen Einheiten, die sich von α,β-ungesättigten $C_{3-6}$-Monocarbonsäuren, ungesättigten $C_{4-6}$-Dicarbonsäuren, kurzkettigen Estern und Amiden dieser Mono- oder Dicarbonsäuren, Hydroxyethylmethacrylat und Vinylpyrrolidon ableiten,

besteht.

17. Verfahren nach Anspruch 16, **dadurch gekennzeichnet, daß** es sich bei dem wärmeschmelzbaren kristallinen Polymer um ein statistisches Copolymer mit ungefähr 10 Gew.-% Einheiten, die sich von Acrylsäure ableiten, und ungefähr 90 Gew.-% Einheiten, die sich von Octadecylmethacrylat ableiten, handelt.

18. Verfahren nach einem der Ansprüche 15 bis 17, **dadurch gekennzeichnet, daß** man das kosmetisch unbedenkliche flüchtige Lösungsmittel mit einem Siedepunkt von weniger als 90°C unter $C_{1-4}$-Alkoholen wie Ethanol oder Isopropanol, Aceton, Methylethylketon, Vinylacetat, Essigsäuremethylester und Dimethoxyethan und Gemischen davon auswählt.

19. Verfahren nach Anspruch 18, **dadurch gekennzeichnet, daß** es sich bei dem flüchtigen Lösungsmittel um Ethanol handelt.

20. Verfahren nach einem der Ansprüche 15 bis 19, **dadurch gekennzeichnet, daß** die Aufhellerlösung außerdem auch noch mindestens ein kosmetisch unbedenkliches Lösungsmittel mit einem Siedepunkt von mehr als 90°C wie Wasser, $C_{6-10}$-Alkane, Diethoxyethan und Essigsäureethylester enthält.

21. Verfahren nach einem der Ansprüche 15 bis 20, **dadurch gekennzeichnet, daß** sich der Anteil des oder der kosmetisch unbedenklichen Lösungsmittel auf mindestens 35 Gew.-% der Aufhellerlösung beläuft.

22. Verfahren nach einem der Ansprüche 15 bis 21, **dadurch gekennzeichnet, daß** die Aufhellerlösung außerdem auch noch ein oder mehrere Farbmittel oder Pigmente enthält.

23. Verfahren nach einem der Ansprüche 15 bis 22, **dadurch gekennzeichnet, daß** die Aufhellerlösung außerdem auch noch ein oder mehrere Additive oder Adjuvantien, die unter Verdickungsmitteln, anionischen, nichtionischen, kationischen oder amphoteren Tensiden, Riechstoffen, Konservierungsmitteln, Sonnenschutzmitteln, Proteinen, Vitaminen, Provitaminen, anionischen, nichtionischen, kationischen oder amphoteren nichtfestigenden Polymeren, mineralischen, pflanzlichen oder synthetischen Ölen, Ceramiden, Pseudoceramiden und gegebenenfalls modifizierten linearen oder cyclischen flüchtigen oder nichtflüchtigen Silikonen ausgewählt sind, enthält.

## Claims

1. Aerosol device for the temporary lightening and/or colouring of the hair, consisting

   - of a container containing an aerosol composition formed from a propellent compound and a liquid phase, and
   - of a means for distributing the said aerosol composition, **characterized in that** the said liquid phase is a lightening solution containing, dissolved in a cosmetically acceptable medium, at least one crystalline hot-melt polymer having a crystal melting point, measured by differential scanning calorimetry, of between 30°C and 80°C, and at least one cosmetically acceptable volatile solvent having a boiling point of less than 90°C, and **in that** the distribution means, which is suitable for obtaining a solids flow rate of less than or equal to 12 mg/s and a wetting power, at 20 cm, of less than or equal to 100 mg/s, is suitable for obtaining, after evaporation of the solvent, a light-coloured opaque deposit.

2. Aerosol device according to Claim 1, **characterized in that** the said distribution means is suitable for obtaining a solids flow rate of between 2 and 12 mg/s.

3. Aerosol device according to Claim 1, **characterized in that** the said distribution means is suitable for obtaining a wetting power of between 10 and 100 mg/s and preferably between 20 and 80 mg/s.

4. Aerosol device according to any one of Claims 1 to 3, **characterized in that** the said distribution means makes it possible to obtain a flow rate of aerosol composition of greater than 200 mg/s, preferably between 200 and 600 mg/s and in particular between 300 and 400 mg/s.

5. Aerosol device according to any one of Claims 1 to 4, **characterized in that** the solids concentration of the aerosol composition (lightening solution + propellent compound) is less than or equal to 25% by weight, preferably between 0.4% and 5% by weight and in particular between 0.6% and 3.25% by weight.

6. Aerosol device according to any one of Claims 1 to 5, **characterized in that** the lightening solution/propellent compound weight ratio is greater than 1 and preferably between 1.2 and 3.

7. Aerosol device according to any one of Claims 1 to 6, **characterized in that** the said hot-melt crystalline polymer consists

   i) of 85% to 98% by weight of hydrophobic units derived from $\alpha,\beta$-ethylenic monomers containing a $C_{12-50}$ and preferably $C_{12-24}$ n-alkyl side chain, forming crystalline homopolymers, and
   ii) of 2% to 15% by weight of hydrophilic units derived from $\alpha,\beta$-unsaturated $C_{3-6}$ monocarboxylic acids, unsaturated $C_{4-6}$ dicarboxylic acids, short-chain esters and amides of these monocarboxylic or dicarboxylic acids, hydroxyethyl methacrylate and vinylpyrrolidone.

8. Aerosol device according to Claim 7, **characterized in that** the said hot-melt crystalline polymer is a random copolymer containing about 10% by weight of units derived from acrylic acid and about 90% by weight of units derived from octadecyl methacrylate.

9. Aerosol device according to any one of Claims 1 to 8, **characterized in that** the concentration of the said crystalline hot-melt polymer is between 0.1% and 35% by weight relative to the total weight of the said lightening solution.

10. Aerosol device according to any one of Claims 1 to 9, **characterized in that** the said cosmetically acceptable volatile solvent having a boiling point of less than 90°C (a) is chosen from $C_{1-4}$ alcohols such as ethanol or isopropanol, acetone, methyl ethyl ketone, vinyl acetate, methyl acetate and dimethoxyethane, and mixtures thereof.

11. Aerosol device according to any one of Claims 1 to 10, **characterized in that** the lightening solution also contains at least one cosmetically acceptable solvent having a boiling point of greater than 90°C, such as water, $C_{6-10}$ alkanes, diethoxyethane or ethyl acetate.

12. Aerosol device according to any one of Claims 1 to 11, **characterized in that** the fraction of the cosmetically acceptable solvent(s) represents at least 35% by weight of the lightening solution.

13. Aerosol device according to any one of Claims 1 to 12, **characterized in that** the lightening solution also contains one or more colorants or pigments.

14. Aerosol device according to any one of Claims 1 to 13, **characterized in that** the said lightening solution also contains one or more additives and adjuvants chosen from thickeners, anionic, nonionic, cationic or amphoteric surfactants, fragrances, preserving agents, sunscreens, proteins, vitamins, provitamins, anionic, nonionic, cationic or amphoteric non-fixing polymers, mineral, plant or synthetic oils, ceramides, pseudoceramides, volatile or non-volatile, linear or cyclic, modified or unmodified silicones.

15. Process for the temporary lightening and/or colouring of the hair, **characterized in that** a lightening solution is applied to dry hair by vaporization, this solution containing, dissolved in a cosmetically acceptable medium, at least one crystalline hot-melt polymer having a crystal melting point, measured by differential scanning calorimetry, of between 30°C and 80°C, and at least one cosmetically acceptable volatile solvent having a boiling point of less than 90°C, so as to form, after evaporation of the solvent, a light-coloured opaque deposit and **in that** the application is carried out using an aerosol device according to one of Claims 1 to 14.

16. Process according to Claim 15, **characterized in that** the said hot-melt crystalline polymer consists

   i) of 85% to 98% by weight of hydrophobic units derived from $\alpha,\beta$-ethylenic monomers containing a $C_{12-50}$ and preferably $C_{12-24}$ n-alkyl side chain, forming crystalline homopolymers, and
   ii) of 2% to 15% by weight of hydrophilic units derived from $\alpha,\beta$-unsaturated $C_{3-6}$ monocarboxylic acids, unsaturated $C_{4-6}$ dicarboxylic acids, short-chain esters or amides of these monocarboxylic or dicarboxylic acids, hydroxyethyl methacrylate and vinylpyrrolidone.

17. Process according to Claim 16, **characterized in that** the said hot-melt crystalline polymer is a random copolymer containing about 10% by weight of units derived from acrylic acid and about 90% by weight of units derived from octadecyl methacrylate.

18. Process according to any one of Claims 15 to 17, **characterized in that** the said cosmetically acceptable volatile solvent having a boiling point of less than 90°C is chosen from $C_{1-4}$ alcohols such as ethanol or isopropanol, acetone, methyl ethyl ketone, vinyl acetate, methyl acetate and dimethoxyethane, and mixtures thereof.

19. Process according to Claim 18, **characterized in**

**that** the said volatile solvent is ethanol.

20. Process according to any one of Claims 15 to 19, **characterized in that** the lightening solution also contains at least one cosmetically acceptable solvent having a boiling point of greater than 90°C, such as water, $C_{6-10}$ alkanes, diethoxyethane or ethyl acetate.

21. Process according to any one of Claims 15 to 20, **characterized in that** the fraction of the cosmetically acceptable solvent(s) represents at least 35% by weight of the lightening solution.

22. Process according to any one of Claims 15 to 21, **characterized in that** the lightening solution also contains one or more colorants or pigments.

23. Process according to any one of Claims 15 to 22, **characterized in that** the said lightening solution also contains one or more additives and adjuvants chosen from thickeners, anionic, nonionic, cationic or amphoteric surfactants, fragrances, preserving agents, sunscreens, proteins, vitamins, provitamins, anionic, nonionic, cationic or amphoteric non-fixing polymers, mineral, plant or synthetic oils, ceramides, pseudoceramides, volatile or non-volatile, linear or cyclic, modified or unmodified silicones.